# EUROPEAN PATENT APPLICATION

(11) **EP 4 067 889 A1**
(43) Date of publication of application: **05.10.2022**
(21) Application number: 21382273.7
(22) Date of filing: 31.03.2021
(51) Int. Cl.: G01N 27/12, G01N 33/543, G01N 33/569

(54) **DETECTION METHOD AND DEVICES**

(71) Applicant: Fundació Eurecat, 08290 Cerdanyola del Vallès (Barcelona) (ES)
(72) Inventor: GARCIA VALLS, Ricard, E-43893 Altafulla (ES); NOGALSKA, Adrianna, E-43007 Tarragona (ES); SHAVEL, Alexey, E-43007 Tarragona (ES)
(74) Representative: ABG Intellectual Property Law, S.L.

(57) **Abstract**

The present invention refers to a membrane electrode assembly (MEA), to a fabrication method of the membrane electrode assembly (MEA), to a cell, electrochemical method for detection of glucosamine, to the use of the membrane electrode assembly (MEA) for the detection of glucosamine, to the use of the cell for the detection of glucosamine or a glycoprotein, and to a non-invasive method for determining a virus infection in a subject.

## Description

### TECHNICAL FIELD

The present invention relates to the field of virus detection. More specifically, the present invention relates to the field of virus detection by electrochemical methods.

### BACKGROUND

Testing, tracking infected people, and tracing their contacts has proved as an effective strategy to reduce the spread of virus. Several methods have been reported for detecting virus, particularly coronavirus, in clinical, research, and public health laboratories. Some tests detect the infection directly by detecting the viral RNA and other tests detect the infection indirectly by detecting the host antibodies.

Uichi et al., (Uichi et al., Sensors 2016, 16, 2045) describes detection systems based on electrochemical biosensors for glycoproteins based on the use of binding domains including antibodies, lectins, phenylboronic acid and molecularly imprinted polymers. In addition, Uichi et al. also discusses biosensors for viruses and bacteria based on their specific interactions with carbohydrates. Unfortunately, some of the glycoprotein sensors cited in Uichi et al., require multi-step measurements for the enhancement of the signals detected. US2015369806 A1 describes a sensor for detecting molecular interactions between a target and a binding domain by electrochemical impedance spectroscopy. Said document uses a lectin as specific domain that specifically binds to a mannose residue, a fucose residue, a sialic acid residue, a glucosamine residue, or a galactosamine residue. Nevertheless binding domains as antibodies, proteins such as lectins or molecularly imprinted polymers require long fabrication process.

Detection methods should have sufficient sensitivity and accuracy to make appropriate clinical decisions rapidly during a pandemic. Therefore, despite the above-mentioned systems, it is desirable to develop alternative detection methods and devices with increased efficiency and overall performance.

### BRIEF DESCRIPTION OF THE INVENTION

The authors of the present invention have developed a membrane electrode assembly (MEA) comprising a proton-exchange membrane coated with functionalized nanoparticles that allows the detection of glucosamine alone or as part of a glycoprotein being part of a virus. The use of the membrane electrode assembly (MEA) of the invention has the advantage of a reduced operative and fabrication cost compared to other techniques of the state of the art (i.e. no binding domains are used in the present invention). In addition, said MEA can be used to detect glucosamine by measuring different electrical parameters such as electrical current or an electrical potential of very small samples within seconds/minutes. The system can also be regenerated and reused.

Thus, a first aspect of the invention is directed to a membrane electrode assembly (MEA) comprising:
- a first diffusion layer comprising two opposite sides;
- metal or metal oxide nanoparticles functionalized with a compound comprising a carboxylic acid group, an imide group, an amine group or a combination thereof; wherein the nanoparticles have an average particle size of between 0.1 and 500 nm; and wherein the nanoparticles comprise an element selected from the group consisting of Au, Fe, Ag, Pt and combinations thereof;
- a proton-exchange membrane comprising two opposite sides;
- a catalyst; and
- a second diffusion layer comprising two opposite sides; wherein the second diffusion layer is a gas diffusion layer;
wherein the metal or metal oxide nanoparticles are coating one side of the proton-exchange membrane;
wherein the catalyst is coating one side of the second diffusion layer;
wherein the proton-exchange membrane is between the first and the second diffusion layer; and
wherein the side of the proton-exchange membrane coated with the metal or metal oxide nanoparticles is in contact with one side of the first diffusion layer and wherein the opposite side of the proton-exchange membrane is in contact with the coated side of the second diffusion layer.

In a particular embodiment, the contact between the proton-exchange membrane and the diffusion layers comprises an electrical contact; preferably is a physical and an electrical contact.

In a second aspect, the invention is directed to a fabrication method of the membrane electrode assembly (MEA) according to the invention, comprising the following steps:
i.providing:
   - a first diffusion layer comprising two opposite sides;
   - metal or metal oxide nanoparticles functionalized with a compound comprising a carboxylic acid group, an imide group, an amine group or a combination thereof; wherein the nanoparticles have an average particle size of between 0.1 and 500 nm; and wherein the nanoparticles comprise an element selected from the group consisting of Fe, Au, Ag, Pt and combinations thereof;
   - a proton-exchange membrane comprising two opposite sides;
   - a catalyst; and
   - a second diffusion layer comprising two opposite sides; wherein the second diffusion layer is a gas diffusion layer;
ii. coating one side of the proton-exchange membrane with the metal or metal oxide nanoparticles and coating one side of the second diffusion layer with the catalyst;
iii. placing the proton-exchange membrane between the first and the second diffusion layer; wherein the side of the proton-exchange membrane coated with the metal or metal oxide nanoparticles is in contact with one side of the first diffusion layer, and wherein the opposite side of the proton-exchange membrane is in contact with the coated side of the second diffusion layer;
iv. joining the first diffusion layer, the proton-exchange membrane and the second diffusion layer; preferably with a binder, more preferably under pressure and/or heat.

In a third aspect, the present invention is directed to membrane electrode assembly (MEA) obtainable by the method of fabrication as defined in the invention.

In another aspect, the present invention is directed to a cell comprising
- a compartment comprising the membrane electrode assembly (MEA) of the invention, and
- at least two current collectors;
wherein the MEA is sandwiched between the current collectors.

In another aspect, the present invention is directed to an electrochemical method for detection of glucosamine in an aqueous solution comprising the following steps:
i. providing
   - a cell as defined in the present invention, and
   - an aqueous solution optionally comprising glucosamine; wherein the aqueous solution is in contact with the nanoparticles of the cell; and
   - an oxygen source; wherein the catalyst of the cell is in contact with the oxygen source;
ii. optionally oxidizing the glucosamine of the aqueous solution to the corresponding oxidized state, while oxygen from the oxygen source is reduced to the corresponding reduced state;
iii. measuring an electrical parameter value of the cell; and
iv. identifying the presence or absence of glucosamine by comparing the electrical parameter value of step (iii) with a reference value;
if the electrical parameter value of step (iii) is larger than the reference value, then glucosamine is present in the aqueous solution.

In another aspect, the present invention is directed to the use of the membrane electrode assembly (MEA) or the cell of the invention for the detection of glucosamine; preferably for the electrochemical detection of glucosamine.

In another aspect, the present invention is directed to an in vitro method for determining a virus infection in a subject comprising:
measuring an electrical parameter value in an aqueous sample fluid from the subject and comparing the electrical parameter value with a reference value by the electrochemical method defined in the present invention,
wherein a subject is identified as having a virus infection if the measured electrical parameter value is larger than the reference value, or
wherein a subject is identified as not having a virus infection if the measured electrical parameter value is equal to or smaller than the reference value.

### FIGURES

Figure 1. UV-VIS absorption spectra of the Au particles in dispersion and (b) a transmission electron microscopy (TEM) micrograph of the Au particles.
Figure 2. Results of amperometric measurements of a glycoprotein peptide at a concentration of 200 mg/mL in PBS.
Figure 3. Exploded view of a cell.
Figure 4. Scheme of the membrane electrode assembly fabrication.

### DETAILED DESCRIPTION OF THE INVENTION

A first aspect of the invention is directed to a membrane electrode assembly (MEA) comprising:
- a first diffusion layer comprising two opposite sides;
- metal or metal oxide nanoparticles functionalized with a compound comprising a carboxylic acid group, an imide group, an amine group or a combination thereof; wherein the nanoparticles have an average particle size of between 0.1 and 500 nm; and wherein the nanoparticles comprise an element selected from the group consisting of Au, Fe, Ag, Pt and combinations thereof;
- a proton-exchange membrane comprising two opposite sides;
- a catalyst; and
- a second diffusion layer comprising two opposite sides; wherein the second diffusion layer is a gas diffusion layer;
wherein the metal or metal oxide nanoparticles are coating one side of the proton-exchange membrane;
wherein the catalyst is coating one side of the second diffusion layer;
wherein the proton-exchange membrane is between the first and the second diffusion layer; and
wherein the side of the proton-exchange membrane coated with the metal or metal oxide nanoparticles is in contact with one side of the first diffusion layer and wherein the opposite side of the proton-exchange membrane is in contact with the coated side of the second diffusion layer.

A particular embodiment is directed to a membrane electrode assembly (MEA) comprising:
- a first diffusion layer comprising two opposite sides;
- metal or metal oxide nanoparticles functionalized with a compound comprising a carboxylic acid group, an imide group, an amine group or a combination thereof; wherein the nanoparticles have an average particle size of between 0.1 and 500 nm; and wherein the nanoparticles comprise an element selected from the group consisting of Fe, Au, Ag, Pt and combinations thereof;
- a proton-exchange membrane comprising two opposite sides;
- a catalyst; and
- a second diffusion layer comprising two opposite sides; wherein the second diffusion layer is a gas diffusion layer;
wherein the metal or metal oxide nanoparticles are coating one of the two opposite sides of the proton-exchange membrane (coated-side);
wherein the catalyst is coating one side of the two opposite sides of the second diffusion layer (coated-side);
wherein the proton-exchange membrane is between the first and the second diffusion layer; and
wherein the coated-side of the proton-exchange membrane is in contact with one side of the first diffusion layer and
wherein the opposite side of the proton-exchange membrane (uncoated side) is in contact with the coated side of the second diffusion layer.

### Diffusion layers

In the context of the present invention the term "diffusion layer" in understood as known by an expert in the field of cells; preferably in fuel cells.

In a particular embodiment, the membrane electrode assembly (MEA) of the invention comprises a first and a second gas diffusion layers.

In a particular embodiment, any of the diffusion layers of the invention is a gas diffusion layer; preferably is a paper gas diffusion layer or a cloth gas diffusion layer.

In an embodiment, the diffusion layer comprises a carbon material; more preferably carbon fibers. In a more particular embodiment, the first diffusion layer is a carbon fiber composite paper.

In another particular embodiment, any of the diffusion layers of the invention has a thickness of between 10 and 400 microns; preferably between 50 and 350 microns; even more preferably about 190 microns.

In a particular embodiment, the second diffusion layer of the invention is coated by a catalyst; preferably wherein the catalyst is a metallic compound; preferably a Pt compound; more preferably is PtB. In a more particular embodiment, the second diffusion layer is a woven carbon cloth coated with a catalyst. In a particular embodiment, the catalyst is coating the second diffusion layer in a concentration of at least 0.1 mg/cm², more preferably of between 0.5 and 10 mg/cm²; even more preferably of between 1 and 6 mg/cm²; even much more preferably of about 4 mg/cm².

In a particular embodiment, the sides of the diffusion layers of the invention have an area of between 0.1 and 100000 cm²; preferably of between 0.5 and 100 cm²; more preferably of between 1 and 50 cm²; even more preferably of between 1 and 20 cm²; much more preferably of about 1, 2, 3, 4, 5, 6, 7, 8, 9 or 10 cm².

### Nanoparticles

The membrane electrode assembly (MEA) of the present invention comprises metal or metal oxide nanoparticles functionalized with a compound comprising a carboxylic acid group, an imide group, an amine group or a combination thereof; wherein the nanoparticles have an average particle size of between 0.1 and 500 nm; and wherein the nanoparticles comprise an element selected from the group consisting of Fe, Au, Ag, and Pt.

In a particular embodiment, the nanoparticles of the present invention are chemically functionalized. In particular they do not comprise a binding domain such as an antibody, lectin, receptor, recombinant protein, glycolipid, glycan or molecularly imprinted polymers, particularly attached to the nanoparticle, more particularly covalently attached to the nanoparticle.

In the context of the present invention, the expression "average particle size" is understood as the arithmetic mean size of the particles calculated from a significant number of measurements of particle sizes, for example over 10, 50 or 100, by any technique known in the art, for example by electron microscopy (transmission electron microscopy or scanning electron microscopy).

In an embodiment, the nanoparticles of the present invention have an average particle size of between 1 and 200 nm; preferably of between 5 and 100 nm.

In a particular embodiment, the nanoparticles of the invention comprise an element selected from the group consisting of Fe, Au, Ag, and Pt; preferably from the group consisting of Fe, Au and Pt; more preferably from Au or Fe.

In a particular embodiment, the nanoparticles of the invention comprise a carboxylic acid group, an imide group, an amine group or a combination thereof; preferably they comprise a carboxylic acid group, an imide group, a secondary amine group or a combination thereof; preferably they comprise a compound selected from the group consisting of N-hydroxysuccinimide (NHS) ester, thioglycolic acid (TGA), aminopropyltriethoxysilane (APTS), citric acid or combinations thereof.

In a more particular embodiment, the nanoparticles of the invention are nanoparticles functionalized with carboxylic acid groups, amine groups, imide groups or with combinations thereof.

In a more particular embodiment, the nanoparticles of the invention are nanoparticles functionalized with carboxylic acid groups; preferably with a compound comprising at least one carboxylic acid; preferably wherein the compound further comprises at least one hydroxyl functional group (-OH) or a thiol (-SH) functional group; more preferably is thioglycolic acid (TGA) or citric acid.

In a more particular embodiment, the nanoparticles of the invention are nanoparticles functionalized with amine groups; preferably with a compound comprising at least one amine group; more preferably a silane compound comprising at least one amine group; preferably at least one secondary amine group; most preferably aminopropyltriethoxysilane (APTS).

In a more particular embodiment, the nanoparticles of the invention are nanoparticles functionalized with imide groups; preferably with a compound comprising at least one imide group; more preferably a compound comprising at least one imide group and an ester group; more preferably a succinimide ester; more preferably a N-hydroxysuccinimide (NHS) ester.

In a particular embodiment, the nanoparticles of the invention are Au nanoparticles functionalized with a compound comprising a carboxylic acid group, an imide group, an amine group or a combination thereof; preferably with a compound comprising a carboxylic acid group or an imide group; more preferably with an N-hydroxysuccinimide (NHS) ester or with thioglycolic acid (TGA).

In a more particular embodiment, the nanoparticles of the invention are gold nanoparticles functionalized with carboxylic acid groups; preferably with a compound comprising a carboxylic acid and a thiol group; more preferably thioglycolic acid (TGA).

In a more particular embodiment, the nanoparticles of the invention are gold nanoparticles functionalized with N-hydroxysuccinimide (NHS) ester; more preferably coated with polyethylene glycol and functionalized with N-hydroxysuccinimide (NHS) ester.

Preferably, the poly(ethylene glycol) coating the nanoparticles of the invention has an average molecular weight of between 10 and 10000 g/mol; preferably an average molecular weight of between 100 and 8000 g/mol; more preferably of between 200 and 6000 g/mol; more preferably of between 205 and 5500 g/mol; more preferably of about 5000 g/mol. In the context of the present invention, the "molecular weight" of the polyethylene glycol may be calculated by any technique known in the art, for example as the z-average molecular weight as measured by high performance liquid chromatography (HPLC) as known in the art; preferably, as the z-average molecular weight calculated from the molecular weight distribution measured by high performance liquid chromatography (HPLC) in g/mol.

In another particular embodiment, the nanoparticles of the invention are iron oxide nanoparticles functionalized with a compound comprising a carboxylic acid group, an imide group, an amine group or a combination thereof; more preferably with a compound comprising a carboxylic acid group or an amine group; preferably with aminopropyltriethoxysilane (APTS) or citric acid.

In a more particular embodiment, the nanoparticles of the invention are magnetite nanoparticles functionalized with amine groups; preferably with secondary amine groups; more preferably with a silane comprising secondary amine groups; more preferably with aminopropyltriethoxysilane (APTS).

In a more particular embodiment, the nanoparticles of the invention are iron oxide nanoparticles functionalized with carboxylic acid groups; preferably with citric acid; more preferably wherein the average particle size of the nanoparticles is between 10 and 30 nm.

In a more particular embodiment, the nanoparticles of the invention are:
gold nanoparticles functionalized with carboxylic acid groups; preferably with a compound comprising a carboxylic acid and a thiol group; more preferably thioglycolic acid (TGA); and/or
gold nanoparticles functionalized with N-hydroxysuccinimide (NHS) ester; more preferably coated with polyethylene glycol and functionalized with N-hydroxysuccinimide (NHS) ester; and/or
magnetite nanoparticles functionalized with amine groups; preferably with secondary amine groups; more preferably with a silane comprising secondary amine groups; more preferably with aminopropyltriethoxysilane (APTS); and /or
iron oxide nanoparticles functionalized with carboxylic acid groups; preferably with citric acid; more preferably wherein the average particle size of the nanoparticles is between 10 and 30 nm.

### Proton-exchange membrane

The membrane electrode assembly (MEA) of the present invention comprises a proton exchange membrane.

In a particular embodiment, the proton exchange membrane comprises a fluorinated polymer; preferably a fluorinated polymer with sulfonic acid sites; preferably a perfluorosulfonic acid (PFSA) polymer.

In more particular embodiment, the proton exchange membrane consist of a fluorinated polymer; preferably a fluorinated polymer with sulfonic acid sites; preferably a sulfonated tetrafluoroethylene based fluoropolymer or perfluorosulfonic acid (PFSA) polymer.

In a particular embodiment, the thickness of the proton exchange membrane of the invention is between 0.050 mm and 0.500 mm; preferably between 0.100 and 0.300 mm; more preferably between 0.150 and 0.200 mm; even more preferably of around 0.180 mm.

In a particular embodiment, the sides of the proton exchange membrane of the invention have an area of between 0.1 and 100000 cm²; preferably of between 0.5 and 100 cm²; more preferably of between 1 and 50 cm²; even more preferably of between 1 and 20 cm²; much more preferably of about 1, 2, 3, 4, 5, 6, 7, 8, 9 or 10 cm².

In an embodiment, the proton exchange membrane of the invention comprises two opposite sides; wherein the metal or metal oxide nanoparticles of the invention are coating one side of the proton-exchange membrane. In a more particular embodiment, the metal or metal oxide nanoparticles of the invention are coating the proton-exchange membrane in an amount of between 0.1 and 10 mg/cm²; preferably between 0.2 and 5 mg/cm²; more preferably between 0.4 and 2 mg/cm²; more preferably between 0.5 and 1 mg/cm². In a more particular embodiment, the nanoparticles coated completely one side of the proton exchange membrane.

In particular, in the membrane electrode assembly (MEA) of the invention, the side coated with the metal or metal oxide nanoparticles of the proton exchange membrane of the invention is in contact with the first diffusion layer, and the opposite side of the proton exchange membrane is in contact with the catalyst coated side of the second diffusion layer.

In a particular embodiment, the membrane electrode assembly (MEA) of the invention further comprises a binder; preferably a polymeric binder; more preferably a fluorinated polymer; preferably a fluorinated polymer with sulfonic acid sites; preferably a perfluorosulfonic acid (PFSA) polymer.

In an embodiment, the first diffusion layer in contact with the side coated with the metal or metal oxide nanoparticles of the proton exchange membrane of the invention is an electrode of the membrane electrode assembly (MEA) of the invention; preferably is an anode.

In another embodiment, the second gas diffusion layer coated with the catalyst of the invention is an electrode of the membrane electrode assembly (MEA) of the invention; preferably is a cathode.

In a particular embodiment, all the layers of the membrane electrode assembly (MEA) of the invention are joined; preferably are joined by a binder.

In a particular embodiment, the membrane electrode assembly (MEA) of the invention has two opposite sides; wherein each side has an area of between 0.1 and 100000 cm²; preferably of between 0.5 and 100 cm²; more preferably of between 1 and 50 cm²; even more preferably of between 1 and 20 cm²; much more preferably of about 1, 2, 3, 4, 5, 6, 7, 8, 9 or 10 cm².

A particular embodiment is directed to a membrane electrode assembly (MEA) consisting of:
- a first diffusion layer comprising two opposite sides;
- metal or metal oxide nanoparticles functionalized with a compound comprising a carboxylic acid group, an imide group, an amine group or a combination thereof; wherein the nanoparticles have an average particle size of between 0.1 and 500 nm; and wherein the nanoparticles comprise an element selected from the group consisting of Fe, Au, Ag, and Pt;
- a proton-exchange membrane comprising two opposite sides;
- a catalyst; and
- a second diffusion layer comprising two opposite sides; wherein the second diffusion layer is a gas diffusion layer;
wherein the metal or metal oxide nanoparticles are coating one side of the proton-exchange membrane;
wherein the catalyst is coating one side of the second diffusion layer;
wherein the proton-exchange membrane is between the first and the second diffusion layer; and
wherein the side of the proton-exchange membrane coated with the metal or metal oxide nanoparticles is in contact with one side of the first diffusion layer and wherein the opposite side of the proton-exchange membrane is in contact with the coated side of the second diffusion layer.

In a particular embodiment, the MEA of the present invention does not comprise binding domains; preferably biological binding domains; more preferably receptors, recombinant proteins, glycolipids, glycans antibodies or lectins; even more preferably antibodies or lectins.

An advantage of the use of functionalized metal or metal oxide nanoparticles on the MEA of the invention is the improvement of the surface area-to-weight ratio and easy modification of the surfaces of said MEA. In addition, the inventors have observed that the use of said particles allows the specific binding of glucosamine to the surface of the proton-exchange membrane. In addition, the MEA of the invention is more robust, cheaper to build and its fabrication requires less steps than a MEA comprising biological binding domains such as antibodies or lectin, among others. Moreover, the MEA of the invention allows a quick detection of glucosamine and, if necessary, the MEA can be regenerated and reused several times, thus reducing the overall cost of the measurements. These advantages also apply to the cell of the invention.

### Fabrication method

A second aspect of the invention is directed to a fabrication method of the membrane electrode assembly (MEA) of the present invention, comprising the following steps:
i.providing:
   - a first diffusion layer comprising two opposite sides;
   - metal or metal oxide nanoparticles functionalized with a compound comprising a carboxylic acid group, an imide group, an amine group or a combination thereof; wherein the nanoparticles have an average particle size of between 0.1 and 500 nm; and wherein the nanoparticles comprise an element selected from the group consisting of Fe, Au, Ag, Pt and combinations thereof;
   - a proton-exchange membrane comprising two opposite sides;
   - a catalyst; and
   - a second diffusion layer comprising two opposite sides; wherein the second diffusion layer is a gas diffusion layer;
ii. coating one side of the proton-exchange membrane with the metal or metal oxide nanoparticles and coating one side of the second diffusion layer with the catalyst;
iii. placing the proton-exchange membrane between the first and the second diffusion layer; wherein one side of the first diffusion layer is in contact with the side of the proton-exchange membrane coated with the metal or metal oxide nanoparticles and wherein the opposite side of the proton-exchange membrane is in contact with the coated side of the second diffusion layer; and
iv. joining the first diffusion layer, the proton-exchange membrane and the second diffusion layer; preferably with a binder, more preferably under pressure and/or heat.

In an embodiment, step (i) of the fabrication method of the invention comprises providing a binder.

In an embodiment, the coating of step (ii) of the fabrication method of the invention is coating by air brushing; preferably at a temperature of between 20 and 40 °C and at a pressure of between 1 and 5 bars; more preferably at a temperature of around 30 °C and at 3 bars.

In an embodiment, step (ii) of the fabrication method of the membrane electrode assembly (MEA) of the present invention, comprises coating one side of the proton-exchange membrane with the metal or metal oxide nanoparticles by air brushing; preferably at a temperature of between 20 and 40 °C and at a pressure of between 1 and 5 bars; more preferably at a temperature of around 30 °C and at 3 bars.

In a particular embodiment, the metal or metal oxide nanoparticles of the fabrication method of the invention are in an ink.

In particular, the ink of the fabrication method of the invention comprises
- the metal or metal oxide nanoparticles of the invention;
- a solvent or a mixture of solvents; preferably water and alcohol; and
- a fluorinated polymer; preferably a fluorinated polymer with sulfonic acid sites; preferably perfluorosulfonic acid (PFSA) polymer.

In a particular embodiment, the fluorinated polymer is in the ink of the invention in between 1 and 10 wt% of the total weight of the ink; preferably between 2 and 8 wt% of the total weight of the ink.

In an embodiment, step (iv) of the fabrication method of the invention is performed under pressure, preferably at between 100 and 600 psi; preferably at between 200 and 500 psi; more preferably around 400 psi.

In an embodiment, step (iv) of the fabrication method of the invention is performed during between 0.1 and 48 h; preferably between 1 and 30 h; more preferably between 2 and 24h.

In another embodiment, step (iv) of the fabrication method of the invention is performed under heat, preferably at temperatures of between 50 and 350 °C; more preferably of between 100 and 300 °C, even more preferably at 150 °C.

In an embodiment, the binder of step (iv) is a a fluorinated polymer; preferably a fluorinated polymer with sulfonic acid sites; preferably a sulfonated tetrafluoroethylene based fluoropolymer or perfluorosulfonic acid (PFSA) polymer.

### MEA

Another aspect of the invention is directed to a membrane electrode assembly (MEA) obtainable by the method of fabrication of the invention in any of its particular embodiments. The membrane electrode assembly (MEA) obtainable by the method of fabrication of the invention, may present all the features and characteristics as the membrane electrode assembly (MEA) described in the first aspect of the invention in any of its embodiments.

### Cell

Another aspect of the invention is directed to a cell comprising:
- a compartment comprising the membrane electrode assembly (MEA) of the invention as defined in any of its particular embodiments, and
- a least two current collectors;
wherein the MEA is sandwiched between the at least two current collectors.

In a particular embodiment, membrane electrode assembly is placed in the cell of the present invention between the current collectors. In another particular embodiment, the membrane electrode assembly is in electrical contact with the at least two current collectors. More particularly, the first diffusion layer of the MEA is in contact with one of the at least two current collectors and the second diffusion layer of the MEA is in contact with the other of the at least two current collectors. Even more particularly, one side of the first diffusion layer of the MEA is in contact with one of the at least two current collectors and a side of the second diffusion layer of the MEA is in contact with the other of the at least two current collectors. In a particular embodiment, the contact comprises an electrical contact; preferably is a physical and an electrical contact.

In a more particular embodiment, the current collectors are made of stainless steel, copper, aluminum or titanium. In a more particular embodiment, the current collectors are in the form of sheets, mesh, or in any configuration of which the current collector allow fluid flow; preferably a mesh. Current collector materials can be selected to be stable at the operating potentials of the cell.

In a particular embodiment the cell of the invention, is a fuel cell; preferably is a passive fuel cell.

In an embodiment, the first diffusion layer in contact with the side coated with the metal or metal oxide nanoparticles of the proton exchange membrane of the invention is an electrode of the membrane electrode assembly (MEA) of the cell of the invention; preferably an anode.

In another embodiment, the second gas diffusion layer coated with the catalyst of the invention is an electrode of the membrane electrode assembly (MEA) of the cell of the invention; preferably a cathode.

In a particular embodiment, the current collectors of the invention have a sheet shape; preferably they comprise holes; more preferably they are metallic.

In another particular embodiment, the current collectors are made of an electrically conductive material; preferably of stainless steel, titanium, gold or platinum; more preferably of stainless steel.

In another particular embodiment, the compartment comprised in the cell of the present invention is configured to be modular, replaceable, scalable and/or independent of the rest of the cell components.

In a particular embodiment, the compartment of the cell of the present invention is made of a plastic material (e.g., polypropylene, polyethylene, etc.) or a coated steel material (e.g., a plastic-coated or rubber-coated, steel tank) to substantially avoid corrosion due to galvanic coupling. In another particular embodiment, the compartment is of any metallic material that avoids corrosion, such as stainless steel, titanium, nickel or nickel alloys; preferably stainless steel, nickel or nickel alloys.

In a particular embodiment, the compartment of the cell of the present invention comprises at least two openings; preferably four openings.

In a particular embodiment, the membrane electrode assembly (MEA) of the invention is placed in the compartment dividing the compartment in two subcompartments; one at each side of the MEA. In a particular embodiment, each of the two subcompartments of the cell is in fluidic communication with one of the two opposite surfaces of the MEA. For example, the subcompartment at the left side of the cell is in fluidic communication with the left side of the MEA (i.e. the chemicals present in a fluid which is contained by the left subcompartment can reach the left side of the MEA) and the right subcompartment is in fluidic communication with the right side of the MEA (i.e. the chemicals present in a fluid contained by the right subcompartment can reach the right side of the MEA, for example if the right subcompartment comprises air, oxygen can reach the right side of the MEA, in particular its surface) .

In the context of the present invention, a "subcompartment" is understood as a compartment that is part of a larger one.

In a particular embodiment, each subcompartment comprises an opening. Preferably, one of said openings is a sample deposition opening.

The cell of the present invention may comprise a controller.

In a particular embodiment, the cell of the present invention may comprise a potenciostat.

In a particular embodiment, the cell of the present invention may comprise a control unit; preferably a control unit adapted to measure volts, coulombs and ampere.

The cell of the present invention may comprise a power/load source. The power/load source is any external electrical device such as an electrical grid, an electric vehicle, a domestic appliance or a sensor, that draws/transfers energy from/to the cell. In general, the power/load source have controllable voltages and/or current supplies or uptakes.

### Detection method

Another aspect of the invention is directed to an electrochemical method for detection of glucosamine in an aqueous solution comprising the following steps:
i. providing
   - a cell as defined in the present invention in any of its particular embodiments,
   - an aqueous solution optionally comprising glucosamine; wherein the aqueous solution is in contact with the nanoparticles of the cell; and
   - an oxygen source; wherein the catalyst of the cell is in contact with the oxygen source;
ii. optionally oxidizing the glucosamine of the aqueous solution to the corresponding oxidized state, while oxygen from the oxygen source is reduced to the corresponding reduced state;
iii. measuring an electrical parameter value of the cell; and
iv. identifying the presence or absence of glucosamine by comparing the electrical parameter value of step (iii) with a reference value;
if the electrical parameter value of step (iii) is larger than the reference value, then glucosamine is present in the aqueous solution.

In an embodiment, glucosamine is understood as (3R,4R,5S)-3-Amino-6-(hydroxymethyl)oxane-2,4,5-triol, derivatives or salts thereof.

In a particular embodiment the glucosamine of the present invention is N-acetylglucosamine; more preferably of N-acetyl-D-glucosamine.

In another particular embodiment, the glucosamine of the present invention is part of a glycoprotein; preferably is part of a spike (S) glycoprotein.

The electrochemical method of the invention can be a method for detection of virus, particularly of any virus containing glycoproteins, more particularly of any virus containing glycoproteins comprising glucosamine.

In another embodiment, the glucosamine of the present invention is part of a virus; preferably part of a coronavirus; more preferably part of alpha, beta, gamma, and delta coronavirus.

In the context of the present invention, the term "glycoprotein" is understood as proteins which contain oligosaccharide chains (glycans) such as glucosamine; preferably wherein said glycans are covalently attached to amino acid side-chains of the protein.

In a particular embodiment, the oxygen source of the present invention is O₂ or air; preferably air.

In a particular embodiment the aqueous solution of the invention further comprises ion conducting compounds; preferably salts, preferably phosphate and/or alkali salts; more preferably comprises sodium or potassium chlorides.

In a particular embodiment the aqueous solution of the invention further comprises a basic compound; preferably an alkali hydroxide; more preferably sodium hydroxide or potassium hydroxide; even much more preferably in a concentration of between 0.01 and 1 M (mol/L); more preferably about 0.1 mol/L. In a particular embodiment the aqueous solution of the invention has a basic pH; preferably has a pH greater than 7, 8 or 9; more preferably has a pH of between 10 and 14; more preferably of between 11 and 13; even more preferably between 11.5 and 12.5; eve much more preferably between 11.75 and 12.25.

In a particular embodiment the aqueous solution of the invention further comprises phosphates; preferably is a phosphate-buffered saline solution as known in the art.

In a particular embodiment the aqueous solution is water, preferably wastewater.

In a particular embodiment the aqueous solution of the invention is a mammal fluid; preferably saliva. In a particular embodiment, the aqueous solution is human saliva.

In a particular embodiment the glucosamine of the aqueous solution is oxidized in the anode of the cell of the present invention; preferably is electroxidized in the anode of the cell of the present invention. In another embodiment, the oxidation of the glucosamine is catalyzed by the nanoparticles of the cell.

In a particular embodiment oxygen from the oxygen source is reduced in the cathode of the cell of the present invention. In another embodiment, the reduction of the oxygen is catalyzed by the catalyst of the cell.

In a particular embodiment the reference value is obtained from a known aqueous solution; preferably from a known aqueous solution comprising a known amount of glucosamine; preferable comprising a known amount of glucosamine from Glycoprotein (S). In a particular embodiment the reference value is obtained from an aqueous solution not containing glucosamine, preferably not containing Glycoprotein (S).

In a particular embodiment, the electrical parameter value of step (iii) is measured between the anode and the cathode of the cell.

In a particular embodiment, the electrical parameter value of step (iii) is an electrical current or an electrical potential.

In a particular embodiment, the electrical parameter value of step (iii) is an electrical current while a known potential is applied between the anode and the cathode; preferably a potential of 0 V.

In particular, the cell or the membrane electrode assembly (MEA) of the present invention might be used for the detection of glucosamine by amperometric measurements, wherein the electrical current occurring due to the oxidation of the glucosamine in the anode and the reduction of oxygen in the cathode is measured, while applying a known potential between the anode and the cathode.

In a particular embodiment, the electrical parameter value of step (iii) is an electrical potential while there is a known electric current between the anode and the cathode of the cell of the invention.

In a particular embodiment, the electrical parameter value of step (iii) is an electrical potential measured in open-circuit potential conditions of the cell of the invention. In the context of the present invention "open-circuit potential conditions" is understood as known in the art.

### Uses

An aspect of the invention is directed to the use of the membrane electrode assembly (MEA) of the invention as defined in any of the particular embodiments, for the detection of glucosamine; preferably for the electrochemical detection of glucosamine; more preferably for the electrochemical detection of glucosamine by amperometric or potentiometric methods.

An aspect of the invention is directed to the use of cell of the invention as defined in any of the particular embodiments, for the detection of glucosamine; preferably for the electrochemical detection of glucosamine; more preferably for the electrochemical detection of glucosamine by amperometric or potentiometric methods.

An aspect of the invention is directed to a method for the detection of glucosamine comprising using the membrane electrode assembly (MEA) of the invention as defined in any of the particular embodiments; preferably for the electrochemical detection of glucosamine; more preferably for the electrochemical detection of glucosamine by amperometric or potentiometric methods.

An aspect of the invention is directed to a method for the detection of glucosamine comprising using the cell of the invention as defined in any of the particular embodiments; preferably for the electrochemical detection of glucosamine; more preferably for the electrochemical detection of glucosamine by amperometric or potentiometric methods.

In a particular embodiment, the glucosamine forms part of a glycoprotein; preferably wherein the glycoprotein is spike (S) glycoprotein; more preferably wherein the S glycoprotein is part of a coronavirus.

### Non-invasive method

In another aspect, the present invention is directed to an in vitro method for determining a virus infection in a subject comprising:
measuring an electrical parameter value in an aqueous sample fluid from the subject and comparing the electrical parameter value with a reference value by the electrochemical method defined in the present invention,
wherein a subject is identified as having a virus infection if the measured electrical parameter value is larger than the reference value, or
wherein a subject is identified as not having a virus infection if the measured electrical parameter value is equal to or smaller than the reference value.

In a particular embodiment, the aqueous solution is an aqueous fluid from a mammal; preferably is saliva.

In a particular embodiment, the non-invasive method for determining a virus infection is an in vitro method.

In a particular embodiment, the non-invasive method for determining a virus infection is a non-invasive method. The term "non-invasive" regarding to the method of the invention relates to an *in vitro* method, i.e., a method which is applied to an aqueous sample fluid previously obtained from the subject under examination and which is able to detect a specific compound in the aqueous sample fluid but which does not require damaging, passing, entering, or penetration through the tissue of the subject.

In a particular embodiment, the non-invasive method for determining a virus infection is an in vitro method.

As used herein the term "determining" and derivations of this term refer to detecting the presence or absence of a virus infection in a subject. According to the present invention, "determining" also includes detecting, evaluating, quantifying, or measuring. This term also includes determining a virus infection in a subject at a time point and determining a virus infection in a subject over time (also denoted herein as "monitoring" and derivations of this term).

The term "subject" or "patient" typically includes humans, but can also include other animals such as, e.g., other primates, rodents, canines, felines, equines, ovines, porcines, and the like. In a preferred embodiment, the subject is a human subject of any sex, race or age.

The term "reference value", as used herein, relates to a predetermined criteria used as a reference for evaluating the values or data obtained from the samples collected from a subject. The reference value or reference level can be an absolute value; a relative value; a value that has an upper or a lower limit; a range of values; an average value; a median value; a mean value; or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. In one embodiment, the reference value is obtained from a healthy subject, particularly from a subject not infected with a virus, more particularly from a subject not infected by a coronavirus.

In another embodiment, the reference value corresponds to an average or mean level determined from a pool of samples obtained from a group of patients who are well documented from the clinical point of view, and who present no disease, particularly who are not suffering a virus infection, particularly not suffering from a coronavirus infection. In said samples, the levels of glucosamine can be determined, for example, by means of the determination of the average levels in a reference population. For example, the reference value can be obtained from identical amounts of a group of at least 2, at least 10, at least 100 to more than 1000 individuals, such that the population is statistically significant. It will be understood that the reference value used for the diagnosis of patients according to the method of the invention is a value obtained from the same type of sample which is being considered in the method.

The term "smaller" in relation to the measured electrical parameter relates to a value detected using the method according to the invention in a sample lower than the reference value. Thus, the measured electrical parameter is considered to be decreased or to be lower or smaller than its reference value when it is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

The term "larger" in relation to the measured electrical parameter relates to a value detected using the method according to the invention in a sample higher than the reference value. Thus, the measured electrical parameter is considered to be increased or to be larger than its reference value when it is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

The term "equal" in relation to the measured electrical parameter relates to a value detected using the method according to the invention in a sample equal or substantially equal to the reference value. Thus, the measured electrical parameter is considered to be equal compared to its reference value when is less than 1%, less than 0.9%, less than 0.8%, less than 0.7%, less than 0.6%, less than 0.5%, less than 0.4%, less than 0.3%, less than 0.2%, less than 0.1%, less than 0.05%, less than 0.01%, less than 0.001%, higher or lower than its reference value.
Preferably the virus infection of the present invention is a coronavirus infection, more preferably a coronavirus infection causing a respiratory syndrome.

The present invention will be described in further detail with reference to the following examples. These examples are for illustrative purposes only and are not intended to limit the scope of the present invention.

### EXAMPLES

The present invention will be described in further detail with reference to the following examples. These examples are for illustrative purposes only and are not intended to limit the scope of the present invention.

### EXAMPLES

### EXAMPLE 1: Nanoparticle synthesis and characterization

Several types of nanoparticles have been used in the present invention as catalyst of glucosamine electrooxidation:

### 1.1. Gold nanoparticles functionalized with thioglycolic acid (TGA)

A solution of 2.2 mM sodium citrate in Milli-Q water (150 mL) was heated for 15 min under vigorous stirring. After boiling, 1 mL of HAuCl₄ (25 mM) was injected in the solution. The color of the solution changed from yellow to bluish gray and then to soft pink in 10 min. The resulting particles (of about 10 nm) are coated with negatively charged citrate ions and hence are dispersed in H₂O. This particles were named Au seeds.

Immediately after the synthesis of the Au seeds and in the same vessel, the reaction was cooled until the temperature of the solution reached 90 °C. Then, 1 mL of sodium citrate (60 mM) and 1 mL of a HAuCl₄ solution (25 mM) were sequentially injected (time delay about 2 min). By repeating the sequential addition of 1 mL of 60 mM sodium citrate and 1 mL of 25 mM HAuCl₄, gold particles of progressively larger sizes were grown. The concentration of each generation of NPs was approximately the same as the original seed particles (3 x 10¹² nanoparticles/mL).

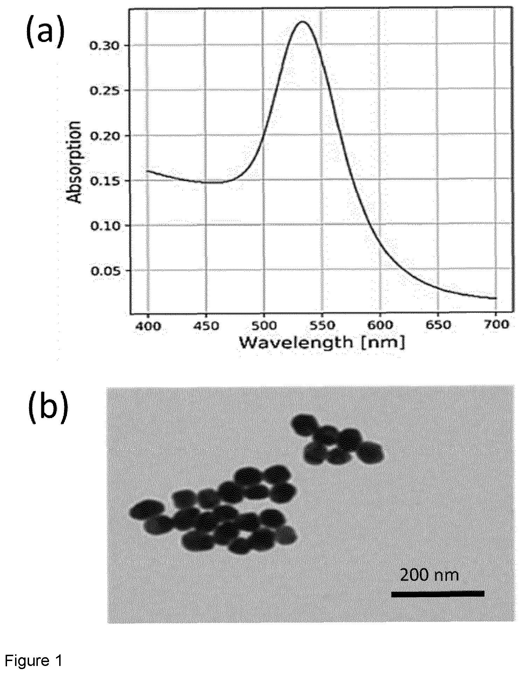

Following the previous process, 60-70 nm Au nanoparticles were synthesized. The particles were characterized by UV-VIS spectroscopy and their size distribution was calculated from a statistically significant number of nanoparticle measurements by transmission electron microscopy (TEM) images. Figure 1 (a) shows a UV-VIS absorption spectra of the Au particles in dispersion and (b) a transmission electron microscopy (TEM) micrograph of the Au particles.

Then, the as synthesized Au nanoparticles were cleaned from excess of the stabilizer (sodium citrate) by subsequent centrifugation and redispersion in a Milli-Q water.

A nanoparticle ink was prepared by mixing Milli-Q water, isopropanol and a Nafion^{®} 117 solution (5 wt.% in a mixture of low aliphatic alcohols and water) with 2.5-5 ml of the Au nanoparticle solution obtained above. A 50 µL of 0.25 M solution of TGA in EtOH was added to the ink to functionalize the Au nanoparticles with thioglycolic acid (TGA). Finally the nanoparticle ink was sonicated (Ultrasons Selecta 3000683, 50/60 kHz, 110 W) during 30 min.

### 1.2. Iron oxide (magnetite) nanoparticles functionalized with -NH₂

The magnetite nanoparticles were synthesized as follows:
Magnetic nanoparticles (MNPs) of magnetite (Fe₃O₄) were synthesized by coprecipitation of iron salts in alkaline medium. An aqueous solution, 50 ml, containing 0.36 M FeCl₂ and 0.72 M FeCl₃ was pumped at 5.0 ml/min (LKB Pump P-1, Pharmacia, Uppsala, Sweden) into 450 ml of 1 M NH₄OH solution under continuous mechanic stirring at room temperature. The obtained black precipitate was separated from the liquid phase using a magnetic field, and then magnetically washed thrice with ultrapure water (MilliQ, Millipore Co., Bedford, MA, USA) and twice with phosphate buffered saline (PBS, 100 mM sodium phosphate, 150 mM NaCl, pH 7.4). All solutions used for the synthesis of MNPs were prepared using MilliQ water. Moreover, a N₂ gas stream was continuously bubbled through all solutions during the process.

The resulting magnetic nanoparticles contained -OH groups on their surface. In order to functionalize them with -NH₂ groups, MNPs (300 mg, dry weight) were incubated with aminopropyltriethoxysilane (APTS) at 70 °C with orbital shaking (200 rpm). After 24 h, they were washed thrice with PBS.

The nitrogen of the amine functional group of the nanoparticles is able to form hydrogen bonds with the hydroxyl groups of glucosamine allowing its adsorption on the surface of the nanoparticles.

### 1.3 Iron oxide nanoparticles functionalized with citric acid

Iron oxide was synthesized according to the article Hui et al. (J. Phys. Chem. C 2008, 112, 30, 11336-11339) with a few modifications. Briefly the synthesis was made as follows:
2 mmol of C₆H₅Na₃O₇·2H₂O (citric acid, trisodium salt dehydrate), 8 mmol of NaOH, and 0.4 mmol of NaSO₄ are mixed in 38 mL of deionized water. The mixture was then heated to 100°C and formed a pellucid solution. 2 mL of 2 M solution of Fe(NO₃)₃ solution was added into the mixture rapidly (equivalent to 0.10 M Fe²⁺ in the alkali solution), and the mixed solution was kept at 100°C for 6h. The solution was then cooled down to room temperature naturally.

Iron oxide nanoparticles were naturally cooled down to room temperature and cleaned by subsequent centrifugation and redispersion in distilled water. Finally, a stable dispersion of the iron oxide nanoparticles in water was obtained. The mean diameter of Iron oxide nanoparticles was around 20 nm and they are giving a stable dispersion in water and keep colloidal stability for at least one month. The NPs can be redispersed in water even after dried into a powder.

### 1.4 Other commercial catalysts tested:

### 1.4.1 Platinum-ruthenium nanoparticles

Commercial Pt-Ru nanoparticles supported on a Woven Carbon Fiber Cloth in a concentration per area of 4 mg/cm² were obtained from The Fuel Cell Store (www.fuelcellstore.com; Product Ref.: W1S100).

### 1.4.2 Gold nanoparticles coated with PEG 5000 and functionalized with N-hydroxysuccinimide (NHS)

Commercial gold nanoparticles coated with polyethylene glycol (PEG) 5000 and functionalized with an N-hydroxysuccinimide (NHS) ester were bought from Sigma Aldrich (Ref.: 900475-1EA).

### EXAMPLE 2 Membrane electrode assembly (MEA)

A nanoparticle ink was prepared by mixing Milli-Q water, isopropanol and a Nafion^{®} 117 solution (5 wt% in a mixture of low aliphatic alcohols and water) with:
- 3-80 mg of the nanoparticles obtained in Example 1.2 or 1.4.2, or
- 2.5-5 ml of the nanoparticle suspension obtained in Example 1.1 or 1.3.

The nanoparticle ink was sonicated (Ultrasons Selecta 3000683, 50/60 kHz, 110 W) during 30 min.

Then, an electrode (anode) for a membrane electrode assembly (MEA) was prepared by depositing the nanoparticle ink described above using air brushing (3 bar, 60°C support heat) on a Nafion^{®}117 membrane to create a catalyst coated membrane (CCM) leading to a nanoparticle concentration in the anode of 0.5-1 mg/cm². The catalyst from Example 1: 1.4.1 was used directly on the Nafion^{®} membrane during MEA preparation step.

A MEA of a fuel cell was prepared using
i) Toray T-060 carbon paper (9 cm² Gas Diffusion Layer (GDL)),
ii) the anode described above (catalyst coated membrane (CCM)),
iii) a commercial cathode (9 cm² cathode Cloth Gas diffusion electrode (GDE) 4 mg/cm² PtB).

The three parts were glued with 100 µL of Nafion^{®} 117 solution (5 wt. %) and pressed for 2 to 24h with 400 psi. The pressure was applied by a manual home-made cell and it was measured with the load compression cell distributed by SENSING, S.L.

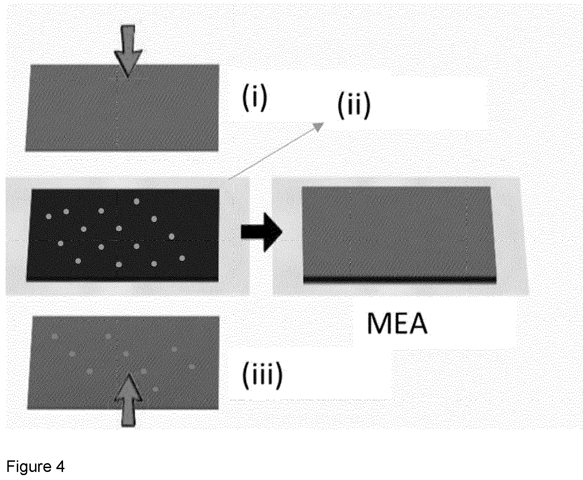

Figure 4 shows a scheme of the membrane electrode assembly fabrication comprising joining a (i) Gas Diffusion Layer (GDL) with a (ii) Catalyst Coated Membrane as anode and a (iii) Gas diffusion electrode as cathode, using as sealing material Nafion^{®}.

### EXAMPLE 3 Electrochemical N-acetylglucosamine and/or glycoprotein detection by amperometric and potentiometric measurements.

Amperometric and potentiometric measurements were performed to detect N-acetylglucosamine and/or glycoprotein in aqueous solution using a Potentiostat (PGSTAT101 Metrohm) to follow the change of signal in time.

Different concentration of 200 mg/ml solutions of N-acetylglucosamine were prepared in aqueous solution of phosphate buffered saline (PBS). PBS is used in biological studies to mimic the saliva conditions as it contains similar salts and it keeps pH stable in around 7. Moreover, PBS works as electrolyte in the studies of glucosamine electrooxidation. The buffer solution was obtained by dissolution of commercial PBS in tablets (pH 7.2-7.6 (1 tablet/200 mL)) in milli-q water.

For the assay, the MEA prepared in Example 2 was assembled together with current collectors, in a 3D-printed plastic cell, where the analyte is supplied at the anode, while the cathode is open to air (O₂). The assembled cell was activated with 0.1% H₂SO₄ during approximately 2 hours. Then the cell was connected to the potentiostat (PGSTAT101. Metrohm) in two-electrode configuration and
i) potential in open-circuit voltage (OCV) mode and
ii) current in amperometric mode (at a potential 0V)
were measured upon the addition of N-acetylglucosamine.

The following results were obtained for the different nanoparticles tested as catalyst for electrooxidation of N-acetylglucosamine:

### Potentiometric measurements (open-circuit voltage (OCV) mode)

The results shown below represent the potentiometric measurements of *membrane electrode assembly* (MEA) prepared with TGA-Au nanoparticles, synthesized as described in Experiment 1.1. In these tests the TGA-Au nanoparticles act as electrocatalysts for oxidation of N-acetylglucosamine in the anode compartment of the fuel cell. All experiments described herein are done in an open-circuit voltage (OCV) mode.

In particular, the results of Table 1 and Table 2 below show a linear response of log(C) to potential (mV) obtained for N-acetylglucosamine in 0.1 M NaOH aqueous solution and in a PBS solution, respectively, when Au-TGA NPs act as catalyst. IN particular, *Table 1* shows the results of the dependence of the OCV potential of a cell comprising the MEA described above on the inverse logarithm of glucosamine concentration in 0.1M NaOH solution. In addition, Table 2 shows the results of the dependence of OCV potential of cell on the inverse logarithm of glucosamine concentration in standard PBS solution.

**Table 1**

| **-Log(C)** | **Potential [mV]** |
|---|---|
| 1.415 | 231 |
| 1.146 | 209 |
| 0.788 | 150 |
| 0.602 | 128 |
| 0.301 | 65 |

**Table 2**

| **-Log(C)** | **Potential [mV]** |
|---|---|
| 2.086 | 125 |
| 1.792 | 108 |
| 1.623 | 95 |

### Amperometric measurements

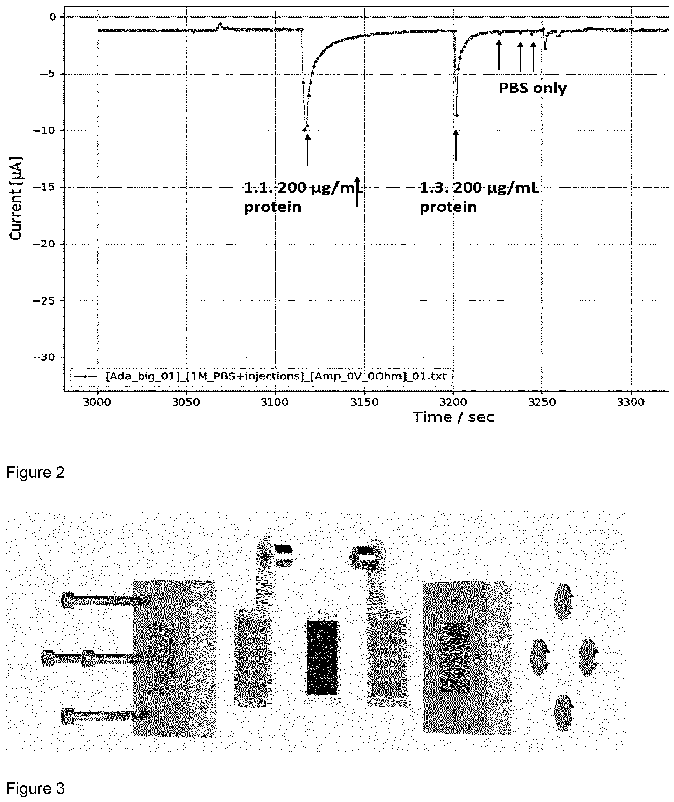

Amperometric measurements results are shown below representing the TGA-Au nanoparticles synthesized as described in Experiment 1.1 and acting as catalyst for electrooxidation of N-acetylglucosamine or a glycoprotein peptide in the anode compartment of MEA as described above and working in amperometric mode
*N-acetylglucosamine.*
   Amperometric measurements of N-acetylglucosamine in PBS showed a signal intensity in a range of 1-100 µA, depending on concentration of the N-acetylglucosamine and the area of MEA used for the tests. The signal loses intensity and returns to the background in a time scale of minutes. In particular, the amperometric measurements for solutions of N-acetylglucosamine in PBS with concentrations 2.5*10⁻²M and 6.1*10⁻⁴M gave a signal of about 3µA in a cell with about 2 cm² MEA.
*Glycoprotein peptide*
   The results of amperometric measurements of a glycoprotein peptide (S1 recombinant protein) at a concentration of 200 mg/mL in PBS are shown in Figure 2. The results show a change in the current when the electrode comprising the TGA-Au nanoparticles is in contact with an aqueous solution of peptide in PBS. However, no change is observed when the solution is containing the PBS buffer only.

A signal in the range of between 50 and 100 µA was obtained for amperometric measurements of the electrooxidation of N-acetylglucosamine in PBS at a concentration 1.6*10⁻²M using magnetite nanoparticles functionalized with APTS (as described in Experiment 1.2) as a catalyst in a cell with a 9 cm² of MEA as described above working in amperometric mode.

Thus, results showed that TGA-Au nanoparticles (as described in Experiment 1.1) and magnetite nanoparticles functionalized with APTS (as described in Experiment 1.2) are able to catalyze the electroxidation of N-acetylglucosamine on the anode of a MEA.

In addition, TGA-Au nanoparticles (as described in Experiment 1.1) are also able to catalyze a Glycoprotein peptide electroxidation in the anode of a MEA.

### EXAMPLE 4: Fuel cell tests

Fuel cells with an anode comprising the nanoparticles described on Example 1 were tested to detect N-acetylglucosamine in aqueous solutions.

A fuel cell comprising the Membrane Electrode Assembly (MEA) described in Example 2 was used for the tests. The fuel cell was a passive fuel cell wherein the anode side had a capacity of 7.2 ml. Ambient air was used as an oxygen source for the cathodic compartment. A Fuel cell Monitor 3.0 (fuel cell store) was used for the electrochemical characterization of the fuel cells in terms of open circuit potential (OCP) [V].

Aqueous solutions of N-acetylglucosamine at different concentrations 0.0-0.8 M were fed to the anode side of the fuel cell. N-acetylglucosamine was dissolved: i) in milli-q water, ii) in a 0.1 M NaOH aqueous solution or iii) in a PBS aqueous solution. In addition, aqueous solutions using ethanol instead of N-acetylglucosamine were also tested.

The catalytic properties of different types of nanoparticles coating the anode were tested using the fuel cell measurements. In particular, the following particles were used:
- Magnetite nanoparticles functionalized with -NH₂ of Example 1, point 1.2.
- Commercial Platinum-ruthenium nanoparticles supported on a carbon cloth in concentration PtRu 4 mg/cm² from Example 1, point 1.4.1. ; and
- Commercial gold nanoparticles functionalized with -NHS from Example 1 point 1.4.2.

For the assay, the MEA prepared in Example 2 was assembled with current collectors, in a 3D-printed plastic cell, where the analyte is supplied at the anode, while the cathode is open to air (as an O₂ source). The assembled cell was activated with 0.1% H₂SO₄ during approximately 2 hours. Then the cell was connected to the Fuel Cell Monitor 3.0. in two-electrode configuration and voltage was measured in Open Circuit Potential (OCP) mode for different concentrations of analyte (mol/I (M)) (N-acetylglucosamine or ethanol). Results showed that not all the nanoparticles were suitable to act as catalysts in the electrooxidation of glucosamine in the anode of the fuel cell.

When using PtRu particles (Example 1, point 1.4.1) as catalyst, signals of about 40 mV were obtained for N-acetylglucosamine dissoved in 0.1 M NaOH, independently of the concentration of N-acetylglucosamine (see Table 3 below). In addition, ethanol solution also gave similar results when tested in the same conditions as N-acetylglucosamine. Therefore, PtRu particles were not selective to glucosamine. The PtRu particles were not tested with N-acetylglucosamine dissolved in water or in PBS solution.

Therefore, results showed that PtRu particles do not allow the selective detection of glucosamine in basic aqueous solutions.

**Table 3**

| Concentration of N-acetylglucosamine in a NaOH solution (0.1M) [M] | OCP [V] |
|---|---|
| 0 | 0.009 |
| 0.1 | 0.040 |
| 0.2 | 0.046 |
| 0.4 | 0.039 |
| 0.8 | 0.035 |

Results for magnetite nanoparticles functionalized with -NH₂ (Example 1, point 1.2.) are showed in Table 4. In summary, N-acetylglucosamine was not detected when dissolved in water at different concentrations in mol/I (M) due to the low conductivity of water. When N-acetylglucosamine is dissolved in a 0.1 M NaOH solution the signal of the hydroxide itself is too high and suppresses that of glucosamine.

Finally, N-acetylglucosamine dissolved in PBS aqueous solutions leads to a signal that rises with the concentration of N-acetylglucosamine. In addition, when tested in the same conditions, ethanol did not give any signal. PBS solutions were used to mimic saliva.

Therefore, results showed that magnetite nanoparticles functionalized with -NH₂ groups allow the selective detection of glucosamine against ethanol in aqueous PBS solutions by its electrooxidation in the anode of a membrane electrode assembly (MEA).

**Table 4**

| N-acetyl glucosamine [M] | OCP [V] in NaOH 0.1M | OCP [V] in PBS | OCP [V] in H₂O |
|---|---|---|---|
| 0 | 0.10 | 0.17 | n.d. |
| 0.1 | 0.13 | - | n.d. |
| 0.3 | 0.12 | 0.25 | n.d. |

Results for gold nanoparticles functionalized with -NHS molecules (Example 1, point 1.4.2.) showed in Table 5 show that signals for N-acetylglucosamine are obtained when said compound is dissolved at a concentration of 0.1 M in a 0.1 M NaOH aqueous solution. Ethanol gave no results when tested in the same conditions.

Therefore, results showed that gold nanoparticles functionalized with -NHS molecules allow the selective detection of glucosamine in a 0.1 M NaOH aqueous solution by the electrooxidation of glucosamine in the anode of a membrane electrode assembly (MEA).

**Table 5**

| N-acetylglucosamine in NaOH (0.1 M) [M] | OCP [V] | pH |
|---|---|---|
| 0 | 0.05 | 12.5 |
| 0.1 | 0.16 | 12 |
| 0.3 | 0.06 | 11.5 |

## Claims

1. A membrane electrode assembly (MEA) comprising:
- a first diffusion layer comprising two opposite sides;
- metal or metal oxide nanoparticles functionalized with a compound comprising a carboxylic acid group, an imide group, an amine group or a combination thereof; wherein the nanoparticles have an average particle size of between 0.1 and 500 nm; and wherein the nanoparticles comprise an element selected from the group consisting of Au, Fe, Ag, Pt and combinations thereof;
- a proton-exchange membrane comprising two opposite sides;
- a catalyst; and
- a second diffusion layer comprising two opposite sides; wherein the second diffusion layer is a gas diffusion layer;
wherein the metal or metal oxide nanoparticles are coating one side of the proton-exchange membrane;
wherein the catalyst is coating one side of the second diffusion layer;
wherein the proton-exchange membrane is between the first and the second diffusion layer; and
wherein the side of the proton-exchange membrane coated with the metal or metal oxide nanoparticles is in contact with one side of the first diffusion layer and wherein the opposite side of the proton-exchange membrane is in contact with the coated side of the second diffusion layer.

2. The membrane electrode assembly (MEA) according to claim 1, wherein the nanoparticles are Au or iron oxide nanoparticles functionalized with a compound comprising a carboxylic acid group, an imide group or an amine group and having an average particle size of between 1 and 200 nm.

3. The membrane electrode assembly (MEA) according to claim 2, wherein the nanoparticles are Au nanoparticles functionalized with thioglycolic acid (TGA) or Au nanoparticles functionalized with N-hydroxysuccinimide (NHS) ester.

4. The membrane electrode assembly (MEA) according to claim 2, wherein the nanoparticles are iron oxide nanoparticles functionalized with aminopropyltriethoxysilane (APTS).

5. The membrane electrode assembly (MEA) according to any one of claims 1 to 4, wherein the proton-exchange membrane is coated with between 0.1 and 10 mg/cm² of nanoparticles.

6. The membrane electrode assembly (MEA) according to any one of claims 1 to 5, wherein the proton-exchange membrane comprises a fluorinated polymer with sulfonic acid sites; preferably a sulfonated tetrafluoroethylene based fluoropolymer-copolymer; and/or
wherein the catalyst is a metallic compound; preferably is a Pt compound; even more preferably is PtB.

7. A fabrication method of the membrane electrode assembly (MEA) according to any one of claims 1 to 5, comprising the following steps:
i.providing:
- a first diffusion layer comprising two opposite sides;
- metal or metal oxide nanoparticles functionalized with a compound comprising a carboxylic acid group, an imide group, an amine group or a combination thereof; wherein the nanoparticles have an average particle size of between 0.1 and 500 nm; and wherein the nanoparticles comprise an element selected from the group consisting of Au, Fe, Ag, Pt and combinations thereof;
- a proton-exchange membrane comprising two opposite sides;
- a catalyst; and
- a second diffusion layer comprising two opposite sides; wherein the second diffusion layer is a gas diffusion layer;
ii. coating one side of the proton-exchange membrane with the metal or metal oxide nanoparticles and coating one side of the second diffusion layer with the catalyst;
iii. placing the proton-exchange membrane between the first and the second diffusion layer; wherein the side of the proton-exchange membrane coated with the metal or metal oxide nanoparticles is in contact with one side of the first diffusion layer and wherein the opposite side of the proton-exchange membrane is in contact with the coated side of the second diffusion layer;
iv.joining the first diffusion layer, the proton-exchange membrane and the second diffusion layer; preferably with a binder, more preferably under pressure and/or heat.

8. A membrane electrode assembly (MEA) obtainable by the method of fabrication as defined in claim 7.

9. A cell comprising
- a compartment comprising
∘ the membrane electrode assembly (M EA) as defined in any one of claims 1 to 6 or 8, and
∘ at least two current collectors;
wherein the MEA is sandwiched between the current collectors.

10. An electrochemical method for detection of glucosamine in an aqueous solution comprising the following steps:
i. providing
- a cell as defined in claim 9, and
- an aqueous solution optionally comprising glucosamine; wherein the aqueous solution is in contact with the nanoparticles of the cell; and
- an oxygen source; wherein the catalyst of the cell is in contact with the oxygen source;
ii. optionally oxidizing the glucosamine of the aqueous solution to the corresponding oxidized state, while oxygen from the oxygen source is reduced to the corresponding reduced state;
iii. measuring an electrical parameter value of the cell; and
iv. identifying the presence or absence of glucosamine by comparing the electrical parameter value of step (iii) with a reference value;
if the electrical parameter value of step (iii) is larger than the reference value, then glucosamine is present in the aqueous solution.

11. The method of claim 10, wherein the glucosamine forms part of a glycoprotein; preferably wherein the glycoprotein is a spike (S) glycoprotein; more preferably wherein the S glycoprotein is part of a coronavirus.

12. The method according to any one of claims 10 or 11, wherein the electrical parameter value measured in step (iii) is an electrical current or an electrical potential.

13. Use of the membrane electrode assembly (MEA) as defined in any of claims 1-6 and 8 or the cell as defined in claim 9 for the detection of glucosamine or a glycoprotein comprising glucosamine; preferably for the electrochemical detection of glucosamine or a glycoprotein comprising glucosamine.

14. An *in vitro* method for determining a virus infection in a subject comprising:
measuring an electrical parameter value in an aqueous sample fluid from the subject and comparing the electrical parameter value with a reference value by the electrochemical method defined in any one of claims 10 to 12,
wherein a subject is identified as having a virus infection if the measured electrical parameter value is larger than the reference value, or
wherein a subject is identified as not having a virus infection if the measured electrical parameter value is equal to or smaller than the reference value.

15. The method for determining a virus infection according to claim 14, wherein the virus infection is a coronavirus infection.
